# EUROPEAN PATENT APPLICATION

(11) **EP 3 404 418 A2**
(43) Date of publication of application: **21.11.2018**
(21) Application number: 18172282.8
(22) Date of filing: 15.05.2018
(51) Int. Cl.: G01N 33/70, C12Q 1/26, C12Q 1/28, C12Q 1/34, G01N 33/558

(54) **A DIAGNOSTIC STRIP FOR DETERMINING THE AMOUNT OF SARCOSINE, CREATININE AND HYDROGEN PEROXIDE IN A BIOLOGICAL OR ENVIRONMENTAL SAMPLE**

(30) Priority: 16.05.2017 CZ 20170272
(71) Applicant: Prevention Medicals s.r.o., 742 13 Studenka - Butovice (CZ)
(72) Inventor: Uhlirová, Dagmar, 602 00 Brno (CZ); Docekalová, Michaela, 696 11 Mutenice (CZ); Stanková, Martina, 664 34 Kurim (CZ); Melichar, Lukás, 691 52 Kostice (CZ); Ruzicka, Josef, 796 01 Prostejov (CZ); Kizek, René, 679 21 Cerná Hora (CZ)
(74) Representative: Rausch Wanischeck-Bergmann Brinkmann

(57) **Abstract**

The present invention relates to a diagnostic strip for an enzymatic test for determining the amount of sarcosine and creatinine based on the formed hydrogen peroxide in a biological sample or in an environmental sample with an option of a visual electronic evaluation by evaluating the intensity of the colour product of the reaction. Zones of an absorbent matrix are applied to the diagnostic strip, where the first reaction zone for the determination of sarcosine contains sarcosine oxidase, sodium salt of 3-(N-ethyl-3-methylaniline) propanesulfonic acid and phenol, and the second reaction zone contains peroxidase and 4-amino-2,3-dimethyl-1-phenyl-3-pyrazole, or the strip in the reaction zone may contain 3,3'-diaminobenzidine, o-Phenylenediamine or ammonium salt of 2.2'-azino-bis(3-ethylbenzothiazoline-6) sulfonic acid. The creatinine diagnostic strip contains in the first reaction zone creatinase, sarcosine oxidase, ascorbate oxidase, catalase, sodium salt of 3-(N-ethyl-3-methylaniline) propanesulfonic acid and phenol and the second reaction zone contains creatininase, peroxidase and 4-amino-2,3-dimethyl-1-phenyl-3-pyrazole or 3,3'-diaminobenzidine or o-Phenylenediamine. The diagnostic strip for determining the amount of hydrogen peroxide contains in the first reaction zone sodium salt of 3-(N-ethyl-3-methylaniline) of propanesulfonic acid or 3,3'-diaminobenzidine or o-Phenylenediamine or ammonium salt of 2.2'-azino-bis(3-ethylbenzothiazoline-6) sulfonic acid and in the second reaction zone contains peroxidase and 4-amino-2,3-dimethyl-l-phenyl-3-pyrazole.

## Description

### Field of the invention

The present invention relates to a diagnostic strip for an enzymatic test for determining the amount of sarcosine and creatinine based on the formed hydrogen peroxide in a biological sample or in an environmental sample.

### Background of the invention

The rapid development of analytical methods has made the development possible of portable ways of identifying selected analytes (substances) both in the environment and in the biological sample [1-3]. To measure the concentration of individual analytes in biological fluids, a number of test devices have been developed. Such devices have been designed to measure, for example, levels of glucose, cholesterol, proteins, ketones, phenylalanine or enzymes in blood, urine, sperm or saliva. Dry reagent strips are used in clinical laboratories, surgeries and households to determine these analytes in body fluid samples. Analysis using enzymatic tests is simple and does not require much time or a specialized operator. The method for the quantitative detection of sarcosine in the urine specimen using chromatography and mass spectrometry is reported in files CN102662013 or CN1026800599. Detection of sarcosine by means of electrophoresis-electrochemiluminescence-based devices is described, for example, in document CN101718746. However, the quantitative routine determination of sarcosine is still challenging in terms of instrumentation, often requiring chemical treatment of the biological sample; furthermore, it is not sufficiently sensitive and accurate.

Detection strips made of plastic material containing multiple zones have been described (part with an immobilized enzyme, chromogenic substrate, reference standard). A significant position is that of the detectors for the inhibition of acetylcholinesterase for capturing combat chemicals, pesticides or organophosphates. Diagnostic test strips serve for rapid semiquantitative analysis of urine, serum or blood. This technology is widely used to determine glucose in particular [4]. It allows easy and rapid testing of clinically significant analytes in urine, serum or blood and to draw conclusions about the presence of various diseases [5]. Proposed tests typically have a long shelf life and do not require additional technical equipment.

However, a strip test for rapid and easy diagnosis of substances related to cancer (sarcosine, haemoglobin), infectious diseases (sarcosine and peroxide) or renal function (creatinine) or a test usable for the detection of hazardous substances from the environment is still missing.

### Literature:

1. Wang, W., et al., Enzymatic hydrolysis device for zero-trans fatty acid, has liquid feeding mechanism whose liquid outlet is connected with liquid inlet of coil pipe, and tank chassis provided with water outlet connected with liquid outlet of coil pipe, GUANGZHOU AOJIAN FENGZE BIOTECHNOLOGY CO (GUAN-Non-standard).
2. Cao, C., et al., Method for qualitative and quantitative detection of protein in dairy product for detecting protein content in food, involves testing pure milk sample and testing electrophoresis fingerprints followed by performing enzymatic hydrolysis, Univ Shanghai Jiaotong (Usjt-C).
3. Ehrenkranz, J.R.L., System for performing enzyme-based diagnostic test to sample e.g. human, has interpretive algorithm to convert detectable signal from detectable label to numerical value for quantification of amount or activity of enzyme in sample, EHRENKRANZ J R L (EHRE-Individual) EHRENKRANZ J R L (EHRE-Individual).
4. Douglas, J., et al., Multilayer reacting testing strip and method for measuring concentration of analyte in sample, I. LIFESCAN, Milpitas, CA, US Editor 1997: USA.
5. Apparatus for testing biological sample e.g. saliva sample for detection of cancer, has test pads arranged side by side, so that outer surface of housing defines window through which portions of front surface of test areas are visible, VIGILANT BIOSCIENCES INC (VIGI-Non-standard).
6. Burg, B., et al., Computer-implemented method for quantifying color change of test medium on diagnostic instrument involves identifying reference samples for medium in the instrument, determining dominant camera-captured color of reference sample/test medium, SCANADU INC (SCAN-Non-standard) SCANADU INC (SCAN-Non-standard) BURG B (BURG-Individual) ZIZI M (ZIZI-Individual) ROWE A A (ROWE-Individual) SMART A (SMAR-Individual) DE BROUWER W (DBRO-Individual) SCANADU INC (SCAN-Non-standard).
7. Wahl, R., O. Cromwell, and H. Fiebig, esting strip for diagnosis of allergies in vitro and use thereof D. MERCK PATENT GMBH, DE, Editor 1997: Německo.
8. TALALAK, Kwanrutai, Julaluk NOIPHUNG, Temsiri SONGJAROEN, Orawon CHAILAPAKUL a Wanida LAIWATTANAPAISAL. A facile low-cost enzymatic paper-based assay for the determination of urine creatinine. Talanta. 2015, 144, 915-921.

### Summary of the invention

The above-mentioned gap is solved by a strip enzymatic test for selected markers for the evaluation of the health condition (renal function, infection, cancer) or the presence of a hazardous substance in the environment, which can be conducted with full blood, serum, plasma, urine, sperm or water. The invention relates to the strip test for the in vitro detection of haemoglobin, sarcosine, creatinine, hydrogen peroxide in a biological sample or in an environmental sample with a visual evaluation of the intensity of the colour product of the reaction, but also with the possibility of electronic evaluation using both a computer and a smartphone.

The subject of the invention is a diagnostic strip for determining the amount of sarcosine in a biological or environmental sample, which consists of a pad on which zones of an absorbent matrix are applied to conduct the test. The strip is 0.4 cm wide and 4.0 cm long. At one end of the strip, a sample zone of 0.5 cm in length is applied, followed by the first reaction zone of 1.0 cm in length, containing sarcosine oxidase with an activity of 1-20 KU/l, sodium salt of 3-(N-ethyl-3-methylaniline) propanesulfonic acid (TOPS) at a concentration of 0.1-2.0 mM and phenol at a concentration of 1-20 mM. The first reaction zone is followed by the second reaction zone of 0.7 cm in length containing peroxidase with an activity of 1-100 KU/l and 4-amino-2,3-dimethyl-1-phenyl-3-pyrazole (AAP) at a concentration of 0.1-2.0 mM. The second reaction zone is followed by a 0.3 cm long detection zone and the detection zone is then followed by a beeswax zone of 1.5 cm in length, forming the other end of the diagnostic strip.

The subject of the invention is also a diagnostic strip for determining the amount of sarcosine of the same embodiment as above, where the strip has a width of 0.4 cm and a length of 4.0 cm. At one end of the strip, a sample zone of 0.5 cm in length is applied, followed by a 2.5 cm-long reaction zone containing sarcosine oxidase with an activity of 1-20 KU/l, 3, 3'-diaminobenzidine (DAB) at a concentration of 1-15 mM, peroxidase with an activity 1-100 KU/l and phenol at a concentration of 1-20 mM. The reaction zone is followed by a 0.3 cm-long detection zone, which is then followed by a beeswax zone (Z) of 0.7 cm in length, forming the other end of the diagnostic strip.

The subject of the invention is also a diagnostic strip for determining the amount of sarcosine of the same embodiment as above, where the strip has a width of 0.4 cm and a length of 4.0 cm. At one end of the strip, a sample zone of 0.5 cm in length is applied, followed by a 2.5 cm-long reaction zone containing sarcosine oxidase with an activity of 1-20 KU/l, o-Phenylenediamine (OPD) at a concentration of 5-25 mM, peroxidase with an activity of 1-100 KU/l and phenol at a concentration of 1-20 mM. The reaction zone is followed by a 0.3 cm-long detection zone, which is then followed by a beeswax zone (Z) of 0.7 cm in length, forming the other end of the diagnostic strip.

In addition, the subject of the invention is a diagnostic strip for determining the amount of sarcosine of the embodiment as above, where the strip has a width of 0.4 cm and a length of 4.0 cm. At one end of the strip, a sample zone of 0.5 cm in length is applied, followed by a 2.5 cm-long reaction zone containing sarcosine oxidase with an activity of 1-20 KU/l, ammonium salt of 2.2'-azino-bis(3-ethylbenzothiazoline-6)sulfonic acid at a concentration of 1-6 mM, peroxidase with an activity of 1-100 KU/l and phenol at a concentration of 1-20 mM. The reaction zone is followed by a 0.3 cm-long detection zone, which is then followed by a beeswax zone (Z) of 0.7 cm in length, forming the other end of the diagnostic strip.

Furthermore, the subject of the invention is a diagnostic strip for determining the amount of creatinine in a biological or environmental sample, which consists of a pad to which zones of an absorbent matrix are applied to conduct the test. The strip is 0.4 cm wide and 4.0 cm long. At one end of the strip, a sample zone of 0.5 cm in length is applied, followed by the first reaction zone of 1.0 cm in length, containing creatinase with an activity of 6-15 KU/l, sarcosine oxidase with an activity of 5-12 KU/l, ascorbate oxidase with an activity of 1-5 KU/l, catalase with an activity of 100-400 KU/l, sodium salt of 3-(N-ethyl-3-methylaniline) propanesulfonic acid (TOPS) at a concentration of 0.3-1.2 mM and phenol at a concentration of 1-15 mM. The first reaction zone is followed by the second reaction zone of 0.7 cm in length containing creatininase with an activity of 50-300 KU/l, peroxidase with an activity of 20-100 KU/l and 4-amino-2,3-dimethyl-1-phenyl-3-pyrazole (AAP) at a concentration of 0.5-4.0 mM. The second reaction zone is followed by a 0.3 cm long detection zone, and the detection zone is then followed by a beeswax zone of 1.5 cm in length, forming the other end of the diagnostic strip.

The first reaction zone of the diagnostic strip for determining creatinine according to another preferred embodiment contains creatinase with an activity of 6-15 KU/l, sarcosine oxidase with an activity of 5-12 KU/l, ascorbate oxidase with an activity of 1-5 KU/l, catalase with an activity of 100-400 KU/l and phenol at a concentration of 1-15 mM, and the second reaction zone contains creatininase with an activity of 50-300 KU/1, peroxidase with an activity of 20-100 KU/l and 3,3'-diaminobenzidine (DAB) with a concentration of 1-15 mM.

The first reaction zone of the diagnostic strip for determining creatinine according to another preferred embodiment contains creatinase with an activity of 6-15 KU/l, sarcosine oxidase with an activity of 5-12 KU/l, ascorbate oxidase with an activity of 1-5 KU/l, catalase with an activity of 100-400 KU/l and phenol at a concentration of 1-15 mM, and the second reaction zone contains creatininase with an activity of 50-300 KU/1, peroxidase with an activity of 20-100 KU/l and o-Phenylenediamine (OPD) with a concentration of 5-25 mM.

The subject of the invention is also a diagnostic strip for determining the amount of hydrogen peroxide in a biological or environmental sample, which consist of a pad on which zones of an absorbent matrix are applied to conduct the test. The strip is 0.4 cm wide and 4.0 cm long. At one end of the strip, a sample zone of 0.5 cm in length is applied, followed by the first reaction zone of 1.0 cm in length, containing sodium salt of 3-(N-ethyl-3-methylaniline) of propanesulfonic acid (TOPS) at a concentration of 0.1-2.0 mM, and the second reaction zone of 0.7 cm in length contains 4-amino-2,3-dimethyl-1-phenyl-3-pyrazole (AAP) at a concentration of 0.1-2.0 mM and peroxidase with an activity of 3-10 KU/l. The second reaction zone is followed by a 0.3 cm long detection zone, and the detection zone is then followed by a beeswax zone of 1.5 cm in length, forming the other end of the diagnostic strip.

The diagnostic strip for determining the amount of hydrogen peroxide of the same embodiment as mentioned above may contain one reaction zone of 2.5 cm in length containing peroxidase with an activity of 3-100 KU/l, 3,3'-diaminobenzidine (DAB) at a concentration of 1 -15 mM.

Another preferred diagnostic strip for determining the amount of hydrogen peroxide of the same embodiment as mentioned above may contain one 2.5 cm-long reaction zone, which contains peroxidase with an activity of 3-100 KU/l, o-Phenylenediamine (OPD) at a concentration of 5-25 mM.

Another preferred diagnostic strip for determining the amount of hydrogen peroxide of the same embodiment as mentioned above may contain one reaction zone containing peroxidase with an activity of 3-100 KU/1, ammonium salt of 2.2'-azino-bis (3-ethylbenzothiazoline-6) sulfonic acid (ABTS) at a concentration of 1-6 mM.

The diagnostic strip pad is preferably made of a plastic film, paper or metal material. The absorbent material is most often made of filtration paper, cellulosic or plastic material. The absorbent carrier is impregnated in a known manner with impregnating solutions. The impregnated and dried absorbent carriers are trimmed into square or rectangular fields which are attached to the strip mat [7].

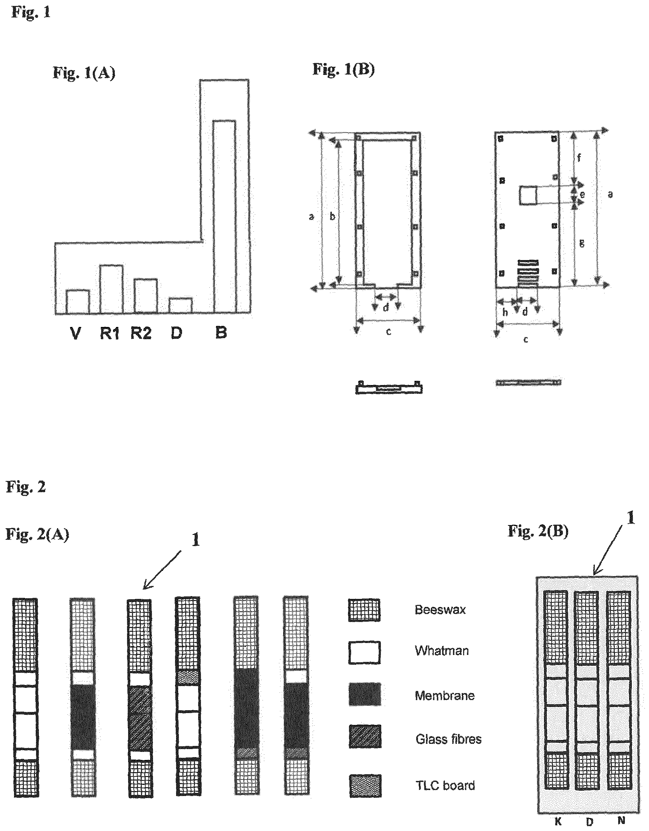

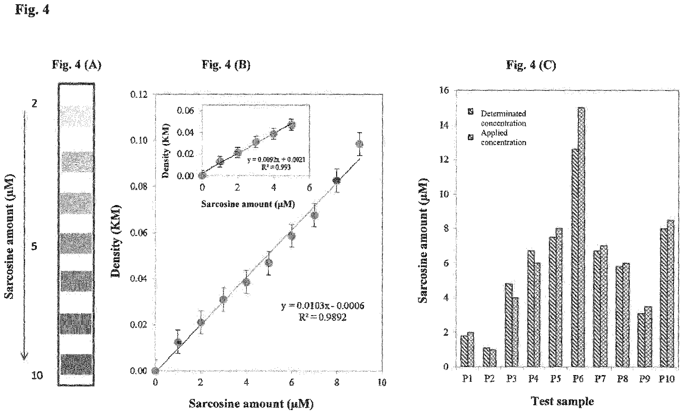

A biological sample suitable for the detection using a diagnostic strip according to the invention is, for example, human urine, serum, plasma or sperm; an environmental sample may be, for example, natural or pool water.
There is a colour intensity scale for each type of a diagnostic strip, determining the intensity of the reaction that has passed and the amount of the analyte (Figure 4-6). During visual evaluation of the quantitative amount of the analyte, the intensity of the product of the colour reaction in the detection zone is compared with the colour intensity of individual detection zones with the control scale of the given colour intensities determining the amount of the test substance in the sample. A typical strip test involves a negative and positive control (Figure 2) [6]. In addition to the control scales of colour intensities of a given colour for a certain analyte, the test system typically includes also test tubes, a test stand, disposable pipettes and suitable detection substrates, such as 4-amino-2,3-dimethyl-1-phenyl-3-pyrazole (AAP), sodium salt of 3-(N-ethyl-3-methylaniline) propanesulfonic acid (TOPS), 3,3'-diaminobenzidine (DAB) and o-phenylenediamine (OPD) and ammonium salt of 2.2'-azino-bis(3-ethylbenzothiazolin-6) sulfonic acid (ABTS).

The invention can be used for easy detection of analytes to determine the amount of sarcosine, creatinine, haemoglobin, hydrogen peroxide in a biological sample in hospitalized patients or for home testing to determine the presence of a given substance in ananalysed sample or a toxic substance in an environmental sample.

### Overview of images:

Fig. 1: (A) Templates prepared using the 3D printing method for the preparation and processing of individual zones of paper detection strips. (B) Design of a simple holder prepared by 3D printing for individual strips; from the left: the lower part, the upper part; a - total length; b - inner part; c - width; d - space for absorption of the sample; e - detection window; f - length above the detection window; g - length under the detection window; h - width for the absorption part.
Fig. 2: (A) Construction diagram of a strip detection test with individual zones. Options of material use for individual zones. (B) Arrangement of our own test system to the form of KDN, where K is a control diagnostic strip, D is a detection strip, N is a negative strip.

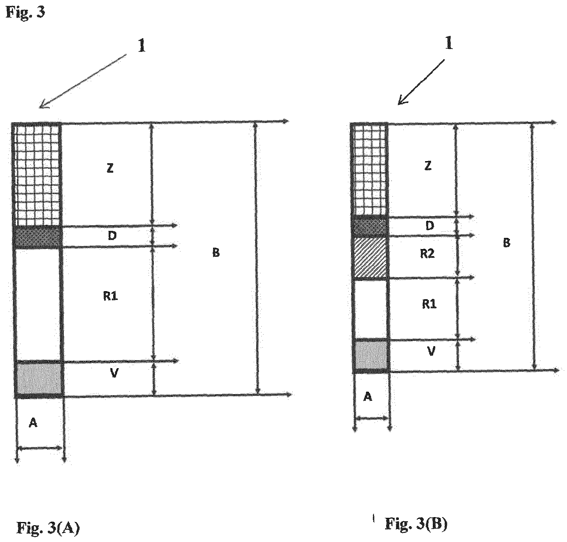

Fig. 3: Detection strip with an arrangement of individual zones, (A) containing two reaction zones; (B) containing one reaction zone; A = 0.4 cm; B = 4.0 cm, V = 0.5 cm; R1 = 1.0 cm; R2 = 0.7 cm; D = 0.3 cm; Z = 1.5 cm.
Fig. 4: Comparative strip to determine the amount of hydrogen peroxide produced in the sample to determine the amount of sarcosine. (A) Colour intensity detection scale used to evaluate the amount of sarcosine in the sample; (B) Dependence of the density of colour reaction on the amount of sarcosine; the linear part of the dependence in the inset (R² = 0.99); (C) Known amount in test samples of artificial urine - the hatched graph, in comparison with the determination of sarcosine concentration in these samples using a detection strip as the applied sarcosine concentration. Average error of determination is 10%.

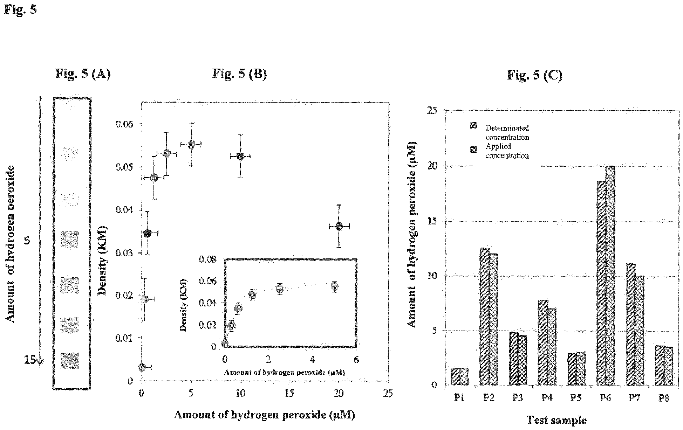

Fig. 5: Detection strip to determine the amount of hydrogen peroxide produced in the sample. (A) Colour detection scale used to evaluate the amount of hydrogen peroxide in the sample; (B) Dependence of the density of colour reaction on the amount of hydrogen peroxide; the linear part of the dependence in the inset (R² = 0.99); (C) Known amount in test samples of artificial urine - the hatched graph, in comparison with the determination of hydrogen peroxide concentration in these samples using a detection strip as the applied hydrogen peroxide concentration. Average error of determination is 8%.

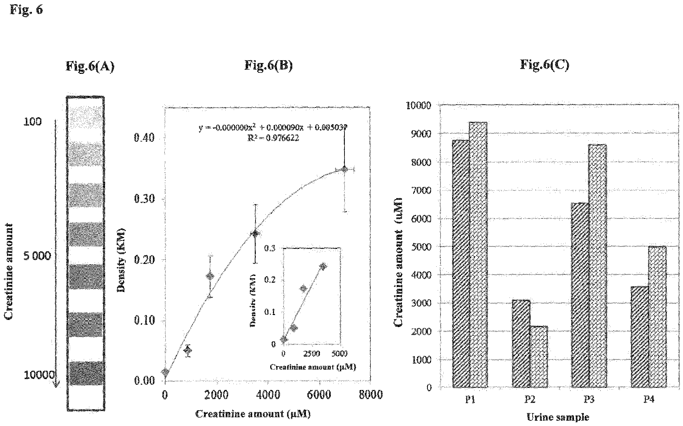

Fig. 6: Detection strip to determine the amount of hydrogen peroxide produced in the sample to determine the creatinine level. (A) Colour detection scale used to evaluate the amount of creatinine in the sample; (B) Dependence of the density of colour reaction on the amount of creatinine; the linear part of the dependence in the inset (R² = 0.95); (C) Application of a detection strip to determine the amount of creatinine in the biological sample (urine), the hatched graph, in comparison with photometric detection (picrate). Average error of determination is 15%.

### Examples of invention execution

### Preparation of a diagnostic strip for the enzymatic determination of sarcosine, creatinine or hydrogen peroxide in the sample

The test was prepared as follows. Diagnostic strip 1 was prepared from the two-sided adhesive pad by cutting off from the adhesive tape (Tesa, Budapest, Hungary); its length was 4.0 cm and width 1.9 cm. On the side of the protective film, a strip of 2.5 cm in length and 0.4 cm in width was cut with a knife. The pad prepared in this way was inserted in a plastic template for the production of strips (Figure 1A). After removal of the protective film outside the defined strip, a thin layer of beeswax was applied. After the wax had dried, the remaining protective film was removed from the defined strip. Rectangles of individual zones from filtration paper Whatman 1 were then prepared (Whatman, GE Healthcare Life Sciences, United Kingdom) with a width of 0.4 cm; for sample zone V with a length of 0.5 cm and for detection zone D with a length of 0.3 cm. Next, a rectangle from the 0.4 cm-wide filtration paper was prepared with a length of 1.0 cm for the first reaction zone R1, a rectangle from the 0.4 cm-wide filtration paper with a length of 0.7 cm for the second reaction zone R2, or a rectangle from the 0.4 cm-wide filtration paper with a length of 2.5 cm for the first reaction zone R1 for the type of the detection strip only with one reaction zone (Figure 3B).

Suitable reagents were applied on the reaction zones for the specific type of the test according to the composition and procedure set in the individual examples. The reagents were allowed to dry. All rectangles from the filtration paper were then gradually glued onto the adhesive pad in the order according to Figure 3 (A) or Figure 3 (B). For better durability and longer shelf life, it is better to lyophilize the rectangular fields after the application of the reagent. Prior to sample testing, sample zone V of prepared diagnostic strips 1 was placed in the vials, initially with several standards of sarcosine, creatinine or hydrogen peroxide of the known quantity, and left in the vial for 5-10 minutes. Alternatively, a test holder can be used (Figure IB). During this time, the sample was rising through capillary action, reacting in the reaction zones and colour-reacting in detection zone D. The reaction was stopped by the beeswax at the end of the strip in zone Z. The result was evaluated in 15 minutes by measuring the intensity of colouring of the detection zone using the Qinslab method (Colour test) (Figure 4-6).

### Example 1

### Determination of sarcosine in a urine sample

For testing, we need a sample of fresh, best morning urine that must be mixed and diagnostic strip 1 with two reaction zones R1 and R2 (Figure 3A) prepared according to the above procedure.

Also prepare a reagent (10 µl) for reaction zone R1 according to the composition and concentrations listed in Table 1:

**Table 1:**

| **Component** | **Concentration** |
|---|---|
| Sarcosine oxidase | 1-20 KU/l |
| Sodium salt of 3-(N-ethyl-3-methylaniline) propanesulfonic acid (TOPS) | 0.1-2.0 mM |
| Phenol | 1-20 mM |

Next, prepare a reagent (5 µl) for reaction zone R2 according to the composition and concentrations listed in Table 2:

**Table 2:**

| **Component** | **Concentration** |
|---|---|
| Peroxidase | 1-100 KU/l |
| 4-amino-2,3-dimethyl-1-phenyl-3-pyrazole (AAP) | 0.1-2.0 mM |

Place the rectangles from the filtration paper prepared according to the procedure described above for reaction zone R1 and reaction zone R2 in a petri dish. Pipette 10 µl of the reagent for zone R1 on the rectangle for zone R1 and 5 µl of the reagent for zone R2 on the rectangle for zone R2. Allow the reagents to dry. Then gradually glue all the rectangles of the filtration paper to the adhesive pad in the order according to Figure 3 (A). For better durability and longer shelf life, it is better to lyophilize the rectangular fields after the application of the reagent.

Place sample zone V of the prepared diagnostic strip with the applied reagents to a vial with a sample; a fast capillary rise occurs. The rise time is 5-10 minutes. During this time, enzymatic reactions occur in reaction zones R1 and R2 and a colour reaction in the detection zone. The enzymatic reaction is stopped in beeswax zone Z at the other end of the strip. The following enzymatic reactions occur in reaction zones R1 and R2:

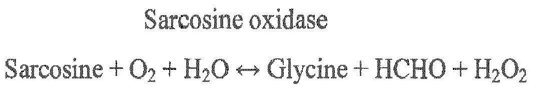

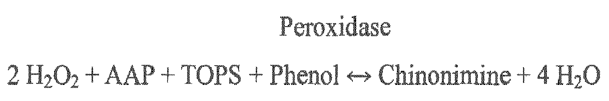

The enzyme sarcosine oxidase decomposes sarcosine to glycine, HCHO and H₂O₂. The produced H₂O₂ reacts with the substrate of 4-amino-2,3-dimethyl-1-phenyl-3-pyrazole (AAP), sodium salt of 3-(N-ethyl-3-methylaniline) propanesulfonic acid (TOPS) and the peroxidase enzyme. A violet-coloured product chinonimine is produced, the colour intensity of which is directly proportional to the concentration of sarcosine in the sample. The amount of sarcosine is proportional to the amount of H₂O₂ produced. Subsequently, the colour of the detection zone is scanned and then evaluated using the Qinslab programme (colour test).

Alternatively, instead of 4-amino-2,3-dimethyl-1-phenyl-3-pyrazole (AAP) and sodium salt of 3-(N-ethyl-3-methylaniline) propanesulfonic acid (TOPS), the following can be used:
a) 3,3'-diaminobenzidine (DAB) at a concentration of 1-15 mM, which forms a brown-coloured product.
   Diagnostic strip 1 only with the first reaction zone R1 of 2.5 cm in length is then used for testing, where the following enzymatic reactions take place: 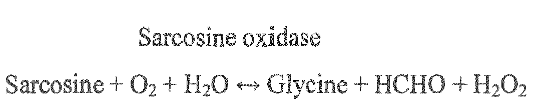 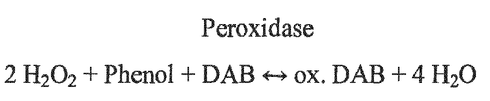
b) o-Phenylenediamine (OPD) at a concentration of 5-25 mM, which forms a yellow-coloured product. Diagnostic strip 1 only with the first reaction zone R1 of 2.5 cm in length is then used for testing, where the following enzymatic reactions take place: 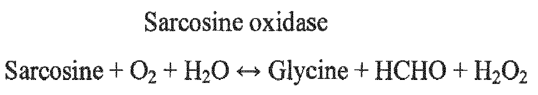 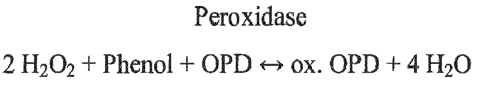
c) Ammonium salt of 2.2'-azino-bis(3-ethylbenzothiazoline-6) sulfonic acid (ABTS) at a concentration of 1-6 mM is used, which forms a green coloured product. Diagnostic strip 1 only with the first reaction zone R1 of 2.5 cm in length is then used for testing, where the following enzymatic reactions take place:

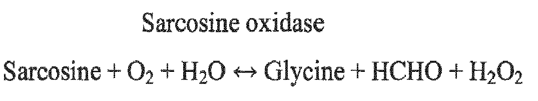

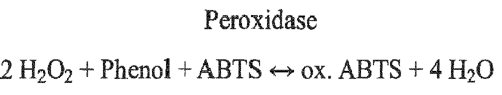

### Example 2

### Determination of hydrogen peroxide in a pool water sample

For testing, we need a sample of pool water and diagnostic strip 1 with one reaction zone R1 prepared according to the above procedure.

Also prepare a reagent (10 µl) for reaction zone R1 according to the composition and concentrations listed in Table 1:

**Table 1:**

| **Component** | **Concentration** |
|---|---|
| Peroxidase | 3-100 KU/l |
| 4-amino-2,3-dimethyl-1-phenyl-3-pyrazole (AAP) | 0.1-2.0 mM |
| Sodium salt of 3-(N-ethyl-3-methylaniline) propanesulfonic acid (TOPS) | 0.1-2.0 mM |

Place the rectangle from the filtration paper prepared according to the procedure described above for reaction zone R1 in a petri dish. Pipette 10 µl of reagent R1 on the rectangle for zone R1. Allow the reagent to dry. Gradually glue all the rectangles of the filtration paper to the adhesive pad in the order according to Figure 3 (B).

Place sample zone V of prepared diagnostic strip 1 with the applied reagent to a vial with a sample; a fast capillary rise occurs. The rise time is 5-10 minutes. During this time, enzymatic reactions in reaction zones R1 and R2 and a colour reaction in detection zone D occur. The following enzymatic reaction occurs in reaction zones R1:

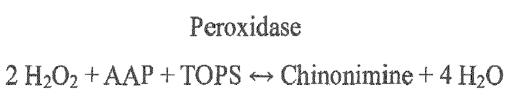

In this reaction, the enzyme peroxidase is important, which decomposes hydrogen peroxide in the presence of the substrate of 4-amino-2,3-dimethyl-1-phenyl-3-pyrazole (AAP) and sodium salt of 3-(N-ethyl-3-methylaniline) propanesulfonic acid (TOPS). After the reaction of the peroxidase enzyme and the substrate, violet-coloured chinonimine is produced, the colour intensity of which is directly proportional to the concentration of H₂O₂. The enzymatic reaction is stopped in beeswax zone Z at the other end of strip 1. Subsequently, the colour of the detection zone is scanned and then evaluated using the Qinslab programme (colour test).

Alternatively, instead of 4-amino-2,3-dimethyl-1-phenyl-3-pyrazole (AAP) and sodium salt of 3-(N-ethyl-3-methylaniline) propanesulfonic acid (TOPS), the following can be used:
a) 3,3'-diaminobenzidine (DAB) at a concentration of 1-15 mM, which forms a brown-coloured product. The following enzymatic reaction then takes place in reaction zone R1 of the strip: 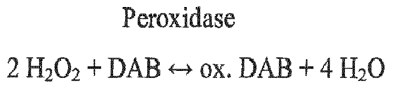
b) o-Phenylenediamine (OPD) at a concentration of 5-25 mM, which forms a yellow-coloured product. The following enzymatic reaction takes place in reaction zone R1 of the strip: 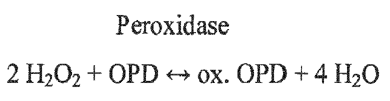
c) Ammonium salt of 2.2'-azino-bis(3-ethylbenzothiazoline-6) sulfonic acid (ABTS) at a concentration of 1-6 mM, which forms a green coloured product. In reaction zone R1 the following enzymatic reaction takes place:

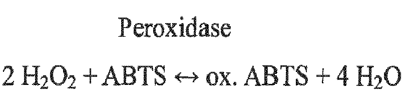

### Example 3

### Determination of creatinine in a plasma sample

For testing, we need a sample of fresh plasma and diagnostic strip 1 with two reaction zones R1 and R2 prepared according to the above procedure.

Also prepare a reagent (10 µl) for reaction zone R1 according to the composition and concentrations listed in Table 1:

**Table 1**

| **Component** | **Concentration** |
|---|---|
| Creatinase | 6-15 KU/l |
| Sarcosine oxidase | 5-12 KU/l |
| Ascorbate oxidase | 1-5 KU/l |
| Catalase | 100-400 KU/l |
| Sodium salt of 3-(N-ethyl-3-methylaniline)-2-hydroxypropanesulfonic acid (TOPS) | 0.3-1.2 mM |
| Phenol | 1-15 mM |

Next, prepare a reagent (5 µl) for reaction zone R2 according to the composition and concentrations listed in Table 2:

**Table 2**

| **Component** | **Concentration** |
|---|---|
| Creatininase | 50-300 KU/l |
| Peroxidase | 20-100 KU/l |
| 4-amino-2,3-dimethyl-1-phenyl-3-pyrazole (AAP) | 0.5-4.0 mM |

Prepare reaction zones R1 and R2 with reagents and complete diagnostic strip 1 as described in Example 1.

Place sample zone V of prepared diagnostic strip 1 with the applied reagents to a vial with a sample. The following enzymatic reactions occur in reaction zones R1 and R2 during the capillary rise:

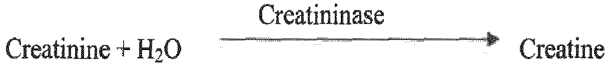

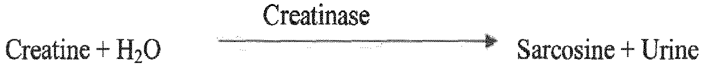

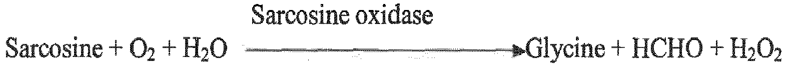

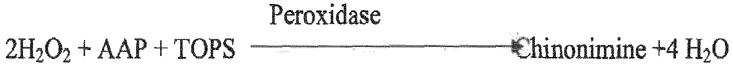

For the determination of creatinine in the urine sample and in plasma, the creatininase enzymes are important, required for the degradation of creatinine, and also the creatinase enzyme, which degrades the creatine produced to sarcosine and urine. Sarcosine cleaves the present enzyme sarcosine oxidase to form peroxide, glycine and formaldehyde. The amount of creatinine is proportional to the amount of H₂O₂ produced. H₂O₂ with the substrate of 4-amino-2,3-dimethyl-1-phenyl-3-pyrazole (AAP), sodium salt of 3-(N-ethyl-3-methylaniline) propanesulfonic acid (TOPS) and the peroxidase enzyme form a violet-coloured product chinonimine, the colour intensity of which is directly proportional to the concentration of creatinine in the sample. The enzymatic reaction is stopped by beeswax at the other end of the strip. Alternatively, the substrate of DAB (3,3'-diaminobenzidine) or OPD (o-Phenylenediamine) can be used, which is added to the reagent for reaction zone R2 as shown in the previous examples.

### Industrial applicability

The test is suitable for the determination of sarcosine, creatinine, hydrogen peroxide in a biological sample, plasma, water, and especially urine. Compared to common procedures (spectrophotometric assay), the determination time is significantly reduced without the need for expensive instrumentation with minimum adjustment. The test result is available in 10 to 15 minutes. The information obtained by this test will allow normalization of further clinical tests and the determination of diagnosis.

### List of reference numerals

- 1: - Diagnostic strip
- A: - Diagnostic strip width
- B: - Diagnostic strip length
- V: - Sample zone
- R1: - The first reaction zone
- R2: - The second reaction zone
- D: - Detection zone
- Z: - Beeswax zone

## Claims

1. A diagnostic strip (1) for determining the amount of sarcosine, creatinine or hydrogen peroxide in a biological or environmental sample, consisting of a pad to which zones of an absorbent matrix are applied to conduct the test, **characterized in that** strip (1) has a width of (A) 0.4 cm and a length of (B) 4,0 cm, and at one end of the strip, sample zone (V) is applied of 0.5 cm in length, followed by reaction zone (R1) of 1.0 cm in length, to which reagent is applied containing sodium salt of 3-(N-ethyl-3-methylaniline) propanesulfonic acid; reaction zone (R1) is followed by reaction zone (R2) of 0.7 cm in length, to which reagent containing peroxidase and 4-amino-2,3-dimethyl-1-phenyl-3-pyrazole is applied; reaction zone (R2) is followed by detection zone (D) of 0.3 cm in length, and detection zone (D) is followed by beeswax zone (Z) of 1.5 cm in length, forming the other end of diagnostic strip (1).

2. A diagnostic strip (1) for determining the amount of sarcosine or hydrogen peroxide according to Claim 1, **characterized in that** reaction zone (R1) contains sodium salt of 3-(N-ethyl-3-methylaniline) propanesulfonic acid at a concentration of 0.1-2.0 mM in the reagent applied and reaction zone (R2) contains peroxidase and 4-amino-2,3-dimethyl-1-phenyl-3-pyrazole at a concentration of 0.1-2.0 mM in the reagent applied.

3. A diagnostic strip (1) for determining the amount of hydrogen peroxide according to Claim 2, **characterized in that** peroxidase in the reagent applied to reaction zone (R2) has an activity of 3-100 KU/l.

4. A diagnostic strip (1) for determining the amount of sarcosine or creatinine according to Claim 1, **characterized in that the** reagent containing sodium salt of 3-(N-ethyl-3-methylaniline) propanesulfonic acid, sarcosine oxidase and phenol is applied to reaction zone (R1).

5. A diagnostic strip (1) for determining the amount of sarcosine according to Claim 4, **characterized in that** the concentration of sodium salt of 3-(N-ethyl-3-methylaniline) propanesulfonic acid in the reagent applied to reaction zone (R1) is 0.1 - 2.0 mM, sarcosine oxidase has an activity of 1-20 mM, phenol has a concentration of 1.0 - 20.0 mM and the activity of peroxidase in the reagent applied to reaction zone (R2) is 1-100 KU/l.

6. A diagnostic strip (1) for determining the amount of creatinine according to Claim 4, **characterized in that** the concentration of sodium salt of 3-(N-ethyl-3-methylaniline) propanesulfonic acid in the reagent applied to reaction zone (R1) is 0.3 - 1.0 mM, sarcosine oxidase has an activity of 5-12 mM, phenol has a concentration of 1.0 - 15.0 mM and the reagent applied to reaction zone (R1) further contains creatinase with an activity of 6-15 KU/l, ascorbate oxidase with an activity of 1-5 KU/l and catalase with an activity of 100-400 KU/1; the activity of peroxidase in the reagent applied to the reaction zone (R2) is 20-100 KU/l; the concentration of 4-amino-2,3-dimethyl-1-phenyl-3-pyrazoleis 0.5-4.0 mM and the reagent applied to reaction zone (R2) further contains creatininase with an activity of 50-300 KU/l.

7. A diagnostic strip (1) for determining the amount of creatinine according to Claim 4, **characterized in that** the activity of sarcosine oxidase in the reagent applied to reaction zone (R1) is 5-12 KU/l, phenol concentration is 1.0-15.0 mM, the activity of creatinase is 6-15 KU/1, the activity of ascorbate oxidase is 1-5 KU/l, the activity of catalase is 100-400 KU/l and the reagent applied to reaction zone (R2) contains peroxidase with an activity of 20-100 KU/l, creatininase with an activity of 50-300 KU/l and 3,3'-diaminobenzidine at a concentration of 1-15 mM in the reagent applied.

8. A diagnostic strip (1) for determining the amount of creatinine according to Claim 7, **characterized in that** the reagent applied to reaction zone (R2) contains peroxidase with an activity of 20-100 KU/1, creatininase with an activity of 50-300 KU/l and o-Phenylenediamine at a concentration of 5-25 mM in the reagent applied.

9. A diagnostic strip (1) for determining the amount of sarcosine or hydrogen peroxide in a biological or environmental sample, consisting of a pad to which zones of an absorbent matrix are applied to conduct the test, **characterized in that** strip (1) has a width of (A) 0.4 cm and a length of (B) 4,0 cm, and at one end of the strip, sample zone (V) is applied of 0.5 cm in length, followed by reaction zone (R1) of 2.5 cm in length, to which reagent is applied containing peroxidase and 3,3'-diaminobenzidine; reaction zone (R1) is followed by detection zone (D) of 0.3 cm in length and detection zone (D) is followed by beeswax zone (Z) of 0.7 cm in length, forming the other end of diagnostic strip (1).

10. A diagnostic strip (1) for determining the amount of hydrogen peroxide according to Claim 9, **characterized in that** the activity of peroxidase in the reagent applied to reaction zone (R1) is 3.0-100.0 KU/l and the concentration of 3,3'-diaminobenzidine is 1.0-15.0 mM.

11. A diagnostic strip (1) for determining the amount of sarcosine according to Claim 10, **characterized in that** the activity of peroxidase in the reagent applied to reaction zone (R1) is 1.0-100.0 KU/l, the concentration of 3,3'-diaminobenzidine is 1.0-15.0 mM and the reagent applied to reaction zone (R1) further contains sarcosine oxidase with an activity of 1.0-20.0 KU/l and phenol at a concentration of 1.0-20.0 mM in the reagent.

12. A diagnostic strip (1) for determining the amount of sarcosine or hydrogen peroxide in a biological or environmental sample, consisting of a pad to which zones of an absorbent matrix are applied to conduct the test, **characterized in that** strip (1) has a width of (A) 0.4 cm and a length of (B) 4,0 cm, and at one end of the strip, sample zone (V) is applied of 0.5 cm in length, followed by reaction zone (R1) of 2.5 cm in length, to which reagent is applied containing peroxidase and o-Phenylenediamine; reaction zone (R1) is followed by detection zone (D) of 0.3 cm in length, and detection zone (D) is followed by beeswax zone (Z) of 0.7 cm in length, forming the other end of diagnostic strip (1).

13. A diagnostic strip (1) for determining the amount of hydrogen peroxide according to Claim 12, **characterized in that** the activity of peroxidase in the reagent applied to reaction zone (R1) is 3.0-100.0 KU/l and the concentration of o-Phenylenediamine in the reagent is 5-25 mM.

14. A diagnostic strip (1) for determining the amount of sarcosine according to Claim 12, **characterized in that** the activity of peroxidase in the reagent applied to reaction zone (R1) is 1.0-100.0 KU/l, the concentration of o-Phenylenediamine is 5-25 mM and the reagent applied to reaction zone (R1) further contains sarcosine oxidase with an activity of 1.0-20.0 KU/l and phenol at a concentration of 1.0-20.0 mM.

15. A diagnostic strip (1) for determining the amount of sarcosine or hydrogen peroxide in a biological or environmental sample, consisting of a pad to which zones of an absorbent matrix are applied to conduct the test, **characterized in that** strip (1) has a width of (A) 0.4 cm and a length of (B) 4,0 cm, and at one end of the strip, sample zone (V) is applied of 0.5 cm in length, followed by reaction zone (R1) of 2.5 cm in length, to which reagent is applied containing peroxidase and ammonium salt of 2.2'-azino-bis(3-ethylbenzothiazoline-6) sulfonic acid; reaction zone (R1) is followed by detection zone (D) of 0.3 cm in length, and detection zone (D) is followed by beeswax zone (Z) of 0.7 cm in length, forming the other end of diagnostic strip (1).

16. A diagnostic strip (1) for determining the amount of hydrogen peroxide according to Claim 15, **characterized in that** the activity of peroxidase in the reagent applied to reaction zone (R1) is 3.0-100.0 KU/l and the concentration of ammonium salt of 2.2'-azino-bis(3-ethylbenzothiazoline-6) sulfonic acid is 1.0-6.0 mM in the reagent.

17. A diagnostic strip (1) for determining the amount of sarcosine according to Claim 15, **characterized in that** the activity of peroxidase in the reagent applied to reaction zone (R1) is 1.0-100.0 KU/l, the concentration of ammonium salt of 2.2'-azino-bis(3-ethylbenzothiazoline-6) sulfonic acid is 1-6.0 mM; the reagent applied to reaction zone (R1) further contains sarcosine oxidase with an activity of 1.0-20.0 KU/l and phenol at a concentration of 1.0-20.0 mM.

18. A diagnostic strip (1) according to Claims 1-17, **characterized in that** the pad of the diagnostic strip is made of a plastic film, paper or metal material.

19. A diagnostic strip (1) according to Claims 1-18, **characterized in that** the absorbent matrix is made of filtration paper, cellulosic or plastic material.

20. A diagnostic strip (1) according to Claims 1-19, **characterized in that** the biological sample is human urine, serum, plasma or sperm.

21. A diagnostic strip (1) according to Claims 1-19, **characterized in that** the environmental sample is pool water.
